(19) **Europäisches Patentamt European Patent Office Office européen des brevets**

(11) **EP 4 180 076 B1**

(12) **FASCICULE DE BREVET EUROPEEN**

(45) Date de publication et mention de la délivrance du brevet:
**11.06.2025 Bulletin 2025/24**

(21) Numéro de dépôt: **22202357.4**

(22) Date de dépôt: **19.10.2022**

(51) Classification Internationale des Brevets (IPC):
***A61M 16/00*** *(2006.01)* ***A61M 39/10*** *(2006.01)*

(52) Classification Coopérative des Brevets (CPC):
**A61M 16/0051; A61M 16/024;** A61M 2016/0039; A61M 2205/15

(54) **DÉTECTION DU DÉBRANCHEMENT DU CIRCUIT PATIENT D'UN VENTILATEUR MÉDICAL À PARTIR DE LA RÉSISTANCE**

ERKENNUNG DER TRENNUNG DES PATIENTENKREISLAUFES EINES MEDIZINISCHEN BEATMUNGSGERÄTES AUS DEM WIDERSTAND

DETECTING PATIENT CIRCUIT DISCONNECT FROM A MEDICAL VENTILATOR FROM RESISTANCE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **15.11.2021 FR 2112043**

(43) Date de publication de la demande:
**17.05.2023 Bulletin 2023/20**

(73) Titulaire: **Air Liquide Medical Systems 92182 Antony Cedex (FR)**

(72) Inventeurs:
• **POMIER, Romain**
  **92182 Antony Cedex (FR)**
• **DERMEL, Marius**
  **92182 Antony Cedex (FR)**
• **JACQUOT, Eric**
  **92182 Antony Cedex (FR)**

(74) Mandataire: **Air Liquide L'Air Liquide S.A. Direction de la Propriété Intellectuelle 75, Quai d'Orsay 75321 Paris Cedex 07 (FR)**

(56) Documents cités:
WO-A1-00/45880        WO-A1-2016/103122
WO-A1-82/01654        US-A- 4 351 344
US-A1- 2015 020 801

**Description**

L'invention concerne une détection du débranchement du circuit patient d'un ventilateur médical alimentant un patient en gaz respiratoire basée sur une estimation de la résistance.

Un ventilateur médical est un appareil d'assistance respiratoire servant à délivrer une aide respiratoire, c'est-à-dire une ventilation artificielle, à un patient souffrant de troubles ou insuffisances respiratoires plus ou moins sévères, pouvant résulter de différentes pathologies ou analogues. Certains patients ayant des pathologies sévères peuvent rester ventilés en milieu hospitalier, pendant plusieurs jours, même plusieurs mois.

Pendant son fonctionnement, le ventilateur médical délivre un gaz respiratoire au patient, par exemple de l'air ou de l'air enrichi en oxygène, via un circuit patient comprenant un ou des conduits servant à véhiculer le gaz, et par ailleurs opère un suivi, i.e. monitorage, de paramètres ventilatoires comme par exemple la pression du gaz, la pression œsophagienne du patient, les volumes de gaz échangés, le débit de gaz... et peut déclencher une alarme sonore ou visuelle pour alerter le personnel soignant en cas de dysfonctionnement du ventilateur ou de problème lié à la ventilation du patient, en particulier en cas de débranchement accidentel du circuit patient.

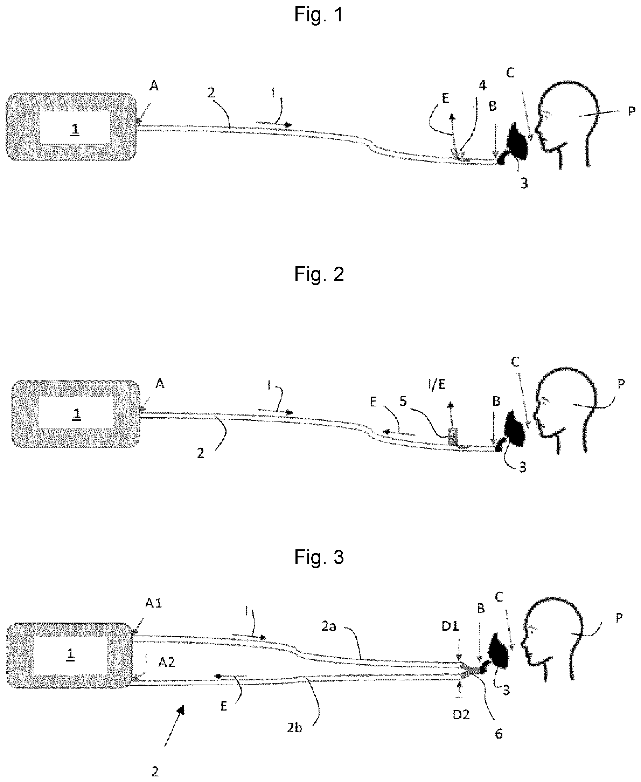

En effet, pendant l'utilisation du ventilateur, il peut survenir une déconnexion ou débranchement accidentel du circuit patient, par exemple au niveau de la liaison entre le ventilateur et le circuit patient, entre le circuit patient et l'interface respiratoire patient alimentant le patient en gaz respiratoire, typiquement un masque respiratoire nasal, buccal ou facial (i.e. bucco-nasal) ou une sonde d'intubation trachéale, ou encore en les branches inspiratoire et/ou expiratoire et la pièce de jonction en Y d'un circuit patient à double branche, comme illustré sur les Fig. 1 à Fig. 3 et détaillé ci-après.

En général, le ventilateur comprend des moyens de détection de débranchement de circuit et des moyens d'alarme servant à détecter tout débranchement du circuit patient et ensuite à prévenir le personnel soignant ou le patient par déclenchement d'une alarme dédiée afin de leur permettre de prendre les mesures adéquates, à savoir une reconnexion des éléments déconnectés.

Or, en pratique, on assiste parfois à des fausses alarmes, c'est-à-dire que le ventilateur détecte par erreur un débranchement alors que tous les éléments sont correctement raccordés.

Il existe plusieurs méthodes de détection d'un débranchement du patient.

Toutefois, ces méthodes ne sont pas idéales car elles nécessitent une prise en compte du type exact de circuit patient utilisé et de ses caractéristiques propres. En effet, un circuit patient comprend des caractéristiques particulières qui sont fonction du patient à traiter, c'est-à-dire qui diffèrent selon que le patient est un adulte, un enfant, un nourrisson...

Or, si l'IHM ou interface homme-machine (e.g. boutons, touches, écran....) d'un ventilateur permet souvent à l'utilisateur d'indiquer la catégorie de patient à traiter (i.e. adulte, enfant ou nourrisson), elle ne permet pas de prendre en compte la référence exacte du circuit patient utilisé, donc de ses particularités, ce qui nuit à une bonne détection des débranchements faute de disposer de toutes les informations nécessaires.

On connait par ailleurs WO-A-82/01654 qui décrit un ventilateur comprenant un capteur de pression servant à déterminer la pression dans le but de suivre la compliance pulmonaire du patient afin de détecter tout risque de pneumothorax ou analogue. Il ne concerne pas la détection d'une déconnexion d'un élément du circuit de gaz.

Le document WO82/01654A1 divulgue un respirateur.

Le problème est dès lors de proposer une méthode de détection améliorée permettant de détecter efficacement les déconnexions réelles d'un (ou des) élément du circuit tout en minimisant les fausses détections de manière à réduire ou éliminer le nombre de déclenchements de fausses alarmes.

Détecter plus efficacement les débranchements d'un ou plusieurs éléments du circuit patient est aussi un enjeu pour le confort du patient. En effet, en cas d'une déconnexion d'un tel débranchement, le ventilateur doit pouvoir le détecter rapidement et passer immédiatement dans un état ou mode spécifique dit « de débranchement » ou « de déconnexion », impliquant par exemple une diminution de la vitesse de la turbine délivrant le gaz, pour éviter ou minimiser les risques de projection de sécrétions, une consommation électrique et de gaz ($O_2$ par exemple) inutile..., pour réduire le bruit acoustique, ... et autres, et ce, dans le but de ne pas nuire au confort du patient.

La solution de l'invention porte alors sur un ventilateur médical, c'est-à-dire un appareil d'assistance respiratoire, comprenant :

- un circuit de gaz interne pour acheminer du gaz comprenant une sortie de gaz,
- au moins un capteur de débit et au moins un capteur de pression agencés sur le circuit de gaz interne pour opérer des mesures de débit et de pression au sein dudit circuit de gaz interne,
- des moyens de pilotage à au moins un microprocesseur configurés pour traiter les mesures de débit et de pression opérées par lesdits capteurs de débit et de pression,
- un circuit patient raccordé fluidiquement à la sortie de gaz du circuit de gaz interne, et
- une interface respiratoire raccordée fluidiquement au circuit patient,

caractérisé en ce que les moyens de pilotage sont configurés pour :

a) déterminer une valeur de résistance $R_{tot}(t)$ à partir d'au moins des mesures de débit et de pression opérées par lesdits capteurs de débit et de pression,

b) comparer la valeur de résistance $R_{tot}(t)$ déterminée à (au moins) une valeur de résistance minimale $R_{min}$ donnée,

c) déterminer un débranchement du circuit patient ou de l'interface respiratoire lorsque la valeur de résistance $R_{tot}(t)$ est inférieure ou égale à la valeur de résistance minimale $R_{min}$ donnée, i.e. $R_{min} \geq R_{tot}(t)$, et

d) déclencher une alarme lorsqu'un débranchement du circuit patient ou de l'interface respiratoire (i.e. l'un, l'autre ou des deux) est déterminé.

D'une façon générale, la résistance ventilatoire reflète ou quantifie la difficulté, pour le gaz respiratoire considéré, tel de l'air, à s'écouler au travers du système de ventilation incluant le circuit de gaz patient, l'interface respiratoire, les connectiques associées et les voies aériennes du patient.

Selon le mode de réalisation considéré, le ventilateur médical de l'invention peut comprendre l'une ou plusieurs des caractéristiques suivantes :

- à l'étape c), les moyens de pilotage sont configurés pour déterminer un débranchement du circuit patient ou de l'interface respiratoire lorsque la valeur de résistance $R_{tot}(t)$ est inférieure à la valeur de résistance minimale $R_{min}$ donnée, c'est-à-dire $R_{min} > R_{tot}(t)$.
- la valeur de résistance minimale $R_{min}$ donnée est préfixé ou mémorisée.
- alternativement, la valeur de résistance minimale $R_{min}$ donnée est fixable ou modifiable par l'utilisateur, typiquement un personnel soignant (i.e. médecin, infirmier ou analogue).
- il comprend des moyens de fixation ou de sélection de résistance minimale pour fixer ou sélectionner une valeur de résistance minimale $R_{min}$ donnée.
- la valeur de résistance minimale $R_{min}$ donnée est comprise entre 0,010 et 0,080 mmH2O/(cL/min), de préférence entre 0,010 et 0,070 mmH2O/(cL/min), par exemple une valeur de Rmin égale à 0,020 mmH2O/(cL/min), 0,012 mmH2O/(cL/min) ou 0,070 mmH2O/(cL/min).
- il comprend des moyens de sélection de catégorie de patient permettant de sélectionner une catégorie de patient choisie parmi adulte, enfant et nourrisson, ladite sélection de catégorie de patient conduisant à une sélection automatique, e.g. prédéfinie et mémorisée, d'une valeur de résistance minimale ($R_{min}$), de préférence comprise entre 0,010 et 0,080 mmH2O/(cL/min).
- les moyens de pilotage sont configurés pour calculer la valeur de résistance $R_{tot}(t)$ à partir de la formule suivante :

$$R_{tot}(t) = R_{patient}(t) + R_{circuit}(t) = \frac{P_{machine}(t)}{Q(t)}$$

où :

- ■ $R_{tot}(t)$ est la résistance totale appliquée au débit gazeux entre la sortie du ventilateur et les alvéoles pulmonaires du patient,
- ■ $R_{patient}(t)$ est la résistance des voies aériennes du patient,
- ■ $R_{circuit}(t)$ est la résistance du circuit de ventilation, i.e. tuyau flexible et autres connectiques,
- ■ $P_{machine}$ est la pression du gaz en sortie du ventilateur,
- ■ $Q(t)$ est le débit de gaz en sortie du ventilateur et fourni au patient.

- les moyens de sélection de catégorie de patient comprennent un organe de sélection manuelle et/ou un écran d'affichage, typiquement une IHM.
- le circuit patient comprend un ou deux conduits de gaz agencés en parallèle, c'est-à-dire un circuit patient à une ou deux branches.
- le circuit patient est relié directement à interface respiratoire ou indirectement via une pièce de jonction ou de raccordement, typiquement une pièce en Y.
- le circuit patient comprend un ou plusieurs conduits souples, i.e. un ou des tuyaux flexibles selon qu'il est à simple branche ou double branche.
- le circuit patient est un circuit à double branche comprenant deux conduits souples agencés en parallèle venant se raccorder à une pièce de jonction, telle une pièce en Y, située entre les deux conduits souples et l'interface patient.
- le circuit patient est un circuit à double branche comprenant une branche inspiratoire et une branche expiratoire.
- la source de gaz est une micro-soufflante motorisée, i.e. comprenant un moteur électrique, aussi appelée soufflante, turbine ou compresseur.
- la micro-soufflante motorisée est configurée pour délivrer un gaz respiratoire de type air ou air enrichi en oxygène (i.e.

mélange air/$O_2$).

- les moyens de pilotage sont configurés pour piloter la micro-soufflante motorisée, en particulier les phases d'accélérations et de freinage/décélération dudit moteur.
- les moyens de pilotage sont configurés pour déterminer un débranchement du circuit patient ou de l'interface respiratoire lorsque la valeur de résistance $R_{tot}(t)$ est inférieure ou égale à la valeur de résistance minimale $R_{min}$ donnée (i.e. $R_{min} \geq R_{tot}(t)$) pendant un temps de confirmation donné, typiquement une durée de moins de 10 secondes, par exemple pendant une durée de 3 à 5 secondes environ.
- le temps de confirmation donné est préfixé ou réglable.
- le circuit de gaz interne comprend au moins un conduit ou passage de gaz.
- lorsque le circuit patient est à simple branche, les moyens de pilotage sont configurés pour déterminer un débranchement du circuit patient ou de l'interface respiratoire situé au niveau de la connexion entre le ventilateur et le circuit patient, et/ou au niveau de la connexion entre le circuit patient et l'interface respiratoire, et/ou au niveau de la région de contact entre l'interface respiratoire et le visage du patient.
- lorsque le circuit patient est à double branche (i.e. une branche inspiratoire et une branche expiratoire), les moyens de pilotage sont configurés pour déterminer un débranchement du circuit patient ou de l'interface respiratoire situé au niveau de la connexion entre le ventilateur et l'une ou l'autre des extrémités amont des deux branches (i.e. branche inspiratoire et branche expiratoire) du circuit patient, et/ou de l'une ou l'autre des connexions entre l'une ou l'autre des extrémités aval des deux branches du circuit et une pièce de jonction, typiquement une pièce en Y, et/ou au niveau de la connexion entre la pièce de jonction (e.g. pièce en Y) et l'interface respiratoire, et/ou au niveau de la région de contact entre l'interface respiratoire et le visage du patient.
- les moyens de pilotage comprennent un ou plusieurs microprocesseurs, typiquement un microcontrôleur.
- les moyens de pilotage comprennent un (ou plusieurs) microprocesseur(s) agencé(s) sur au moins une carte électronique.
- les moyens de pilotage comprennent un (ou plusieurs) microprocesseur(s) mettant en œuvre au moins un algorithme.
- il comprend en outre des moyens d'alimentation électrique alimentant en courant électrique le ou les composants nécessitant de l'électricité pour fonctionner, notamment les moyens de pilotage.
- les moyens d'alimentation électrique comprennent des moyens de raccordement au secteur (110/220V), tels que câble(s) électrique(s) et/ou prise secteur.
- il comprend en outre une carcasse ou coque externe rigide, par exemple en polymère ou en métal.
- il comprend en outre des moyens de mémorisation, par exemple une mémoire informatique ou analogue, pour mémoriser, i.e. stocker, des données, des valeurs, des informations ou autres.
- les moyens de mémorisation sont configurés pour mémoriser au moins une valeur de résistance minimale $R_{min}$
- les moyens de pilotage, typiquement le processeur, sont configurés pour déclencher une alarme sonore et/ou visuelle en cas de débranchement du circuit, c'est-à-dire quand un débranchement est détecté.
- l'alarme visuelle comprend un message d'alarme s'affichant sur l'écran d'affichage, l'allumage d'un dispositif d'alerte lumineux, tels une ou des LEDs par exemple.
- l'alarme sonore comprend l'émission d'un signal sonore audible par l'utilisateur, par exemple diffusé via un haut-parleur du ventilateur ou analogue.

La technologie va maintenant être mieux comprise grâce à la description détaillée suivante, faite à titre illustratif mais non limitatif, en référence aux figures annexées parmi lesquelles :

Fig. 1 schématise les sites de débranchement d'un circuit patient à simple branche à valve expiratoire,
Fig. 2 schématise les sites de débranchement d'un circuit patient à simple branche dit « à fuites »,
Fig. 3 schématise les sites de débranchement d'un circuit patient à double branche, et
Fig. 4 schématise un ventilateur médical selon l'invention.

Fig. 1 et Fig. 2 schématisent différents débranchements possibles d'un circuit patient 2 à simple branche, e.g. un conduit flexible ou analogue, reliant fluidiquement un ventilateur médical 1, c'est-à-dire un appareil d'assistance respiratoire, à une interface respiratoire 3, tel un masque respiratoire ou une sonde trachéale. Le circuit patient 2 permet d'acheminer le gaz respiratoire, tel de l'air ou un mélange air/O2, délivré par le ventilateur médical 1 jusqu'au patient P alors de l'administrer par inhalation au patient P, pendant ses phases inspiratoires.
Sur Fig. 1, la branche unique du circuit patient 2 comprend une valve expiratoire 4 servant à évacuer vers l'atmosphère, le gaz riche en $CO_2$ expiré par le patient P pendant ses phases expiratoires, alors que sur Fig. 2, la branche unique du circuit patient 2 comprend un orifice de fuite 5 relié à l'atmosphère. Les flèches I et E donnent le sens de circulation des gaz dans le circuit patient 2, pendant les phases inspiratoires (I) et phases expiratoires (E), y compris au travers de la valve expiratoire 4 et de l'orifice de fuite 5. Alternativement, l'orifice de fuite 5 relié à l'atmosphère peut aussi être situé sur l'interface respiratoire 3, tel un masque respiratoire.

Dans ces deux modes de réalisation, les débranchements du circuit patient 2 à simple branche peuvent se produire au niveau de la connexion A entre ventilateur 1 et circuit 2, et/ou au niveau de la connexion B entre circuit 2 et interface respiratoire 3 et/ou au niveau de la région de contact C entre interface respiratoire 3 et visage du patient P.

Fig. 3 est analogue aux Fig. 1 et Fig. 2, à l'exception du fait qu'elle schématise les différents débranchements possibles d'un circuit patient 2 à double branche 2a, 2b, e.g. deux conduits flexibles agencés en parallèle ou analogue, reliant fluidiquement le ventilateur médical 1 l'interface respiratoire 3, tel un masque respiratoire, une sonde trachéale ou autre, par l'intermédiaire d'une pièce de jonction 6 en forme de Y, généralement appelée « pièce en Y ». Le circuit patient 2 comprend une branche inspiratoire 2a acheminant le gaz depuis le ventilateur 1 vers le patient P (sens de la flèche I) et une branche expiratoire 2a acheminant le gaz riche en $CO_2$ expiré par le patient P jusqu'au ventilateur 1 (sens de la flèche E).

Dans ce cas, les débranchements du circuit patient 2 peuvent se produire au niveau des connexions A1, A2 entre ventilateur 1 et les extrémités amont des branches 2a, 2b du circuit 2, et/ou des connexions D1, D2 entre les extrémités aval des branches 2a, 2b du circuit 2 et la pièce en Y 6, et/ou au niveau de la connexion B entre la pièce en Y 6 elle-même et l'interface respiratoire et/ou au niveau de la région de contact C entre interface respiratoire 3 et visage du patient P.

Il est indispensable de pouvoir détecter de tels débranchements du circuit patient 2 du ventilateur médical 1. Pour ce faire, le ventilateur médical 1 comprend des moyens d'alarme incluant une alarme de surveillance de l'état de débranchement du circuit 2. Lorsque celle-ci détecte une déconnexion du circuit 2, elle se lève et prévient le patient ou le personnel médical.

De façon générale, la détection d'un débranchement par le ventilateur 1 est un compromis entre la détection d'un plus grand nombre de bonnes détections, c'est-à-dire que le débranchement est détecté par le ventilateur dès déconnexion d'un élément du chemin d'air à l'une ou l'autre des différentes localisations possibles, comme expliqué ci-avant, et la minimisation des fausses détections, c'est-à-dire que le ventilateur détecte un débranchement alors que tous les éléments sont correctement raccordés. A l'inverse, il est aussi important de pouvoir détecter une reconnexion de tous les éléments constituant le chemin d'air entre le ventilateur 1 et le patient.

Selon la présente technologie, pour répondre à ce problème de détection efficace de débranchement, le ventilateur médical est configuré pour opérer une estimation de la résistance, comme expliqué ci-après.

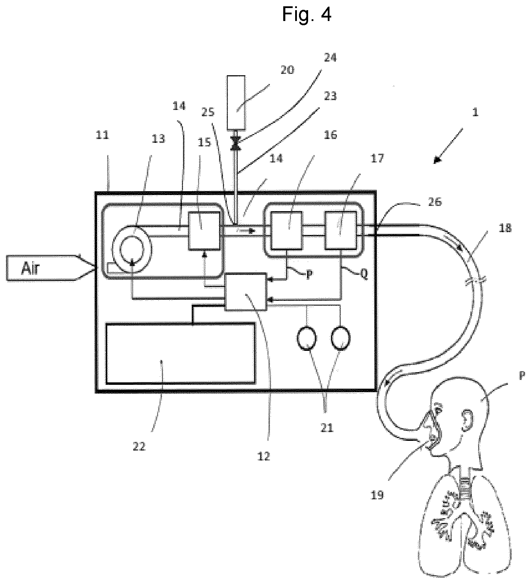

La Fig. 4 schématise un mode de réalisation d'un ventilateur médical 1 ou appareil d'assistance respiratoire permettant de réaliser une détection efficace de débranchement du circuit patient 18 basée sur la résistance.

Le ventilateur médical 1 de l'invention comprend une carcasse ou coque externe 12 dans laquelle est agencée une source de gaz 3, à savoir ici une micro-soufflante motorisée, c'est-à-dire équipée d'un moteur électrique animant une roue à ailettes, délivrant ici un flux d'air (teneur en oxygène 21% en vol.) dans un chemin de gaz, i.e. un circuit de gaz 14 interne, en communication fluidique avec la sortie d'air de la micro-soufflante 13.

Le circuit de gaz interne 14 comprend un ou des conduits ou passages de gaz, ou analogue, configuré pour convoyer le gaz au sein de la carcasse 11 du ventilateur 1 jusqu'à une sortie de gaz 26, aussi appelée sortie ventilateur.

Selon un autre mode de réalisation (non montré), la source de gaz 13 peut être une source externe du ventilateur 1, tel une alimentation en air comprimé, par exemple un conduit souple relié à une prise de distribution murale de gaz ou à un récipient de gaz sous pression, telle une bouteille de gaz sous pression.

L'air provenant de la source de gaz 13 est acheminé par le circuit de gaz interne 14 jusqu'à un patient P par l'intermédiaire d'un circuit patient 18, tel un conduit de gaz flexible, par exemple un (ou des) tuyaux souples en polymère, auquel il est administré au moyen d'une interface respiratoire 19, tel un masque nasal ou facial. Le circuit patient 18 vient se raccorder fluidiquement à la sortie de gaz 26 du ventilateur 1.

Le circuit patient 18 peut être à simple branche, comme illustré sur Fig. 4, ou à double branche (i.e. une branche inspiratoire et une branche expiratoire), comme celui illustré sur Fig. 3.

Un premier capteur de débit 15, un capteur de pression 16 et un second capteur de débit 17 sont agencés, en série, sur le circuit interne de gaz 14, en aval de la micro-soufflante 13, pour y opérer des mesures de pression P et de débit Q du gaz qui y circule. Le capteur de pression 16 est agencé entre le premier capteur de débit 15 et le second capteur de débit 17.

Des moyens de pilotage 12, c'est-à-dire une unité de traitement et de commande, comprenant typiquement une carte électronique comprenant un (ou des) microprocesseur, tel un microcontrôleur, mettant en œuvre au moins un algorithme, reçoit et traite les mesures (i.e. signaux) opérées par les capteurs de pression 16 et de débit 15, 17.

Les moyens de pilotage 12 commandent ici l'alimentation en gaz, c'est-à-dire ici la source de gaz 13 de type micro-soufflante motorisée, délivrant le flux d'air dans le circuit de gaz 14 de manière à délivrer un débit et/ou une pression de gaz selon les modalités du mode de ventilation sélectionné par le médecin, lesquels modes et modalités sont par exemple renseignées au moyen de boutons de réglages 21 et/ou d'un écran 22, de préférence tactile, formant une IHM ou interface homme-machine.

Les composants du ventilateur 1, notamment ici la micro-soufflante 13 motorisée, les capteurs de pression et de débit 15-17, au moins une partie du circuit de gaz 14 et les moyens de pilotage 12 sont agencés dans la carcasse externe 11 de l'appareil.

Par ailleurs, une seconde source de gaz 20 contenant de l'oxygène ou « gaz riche en oxygène », à savoir ici une bouteille d'oxygène ou une canalisation d'oxygène, est reliée fluidiquement au circuit de gaz 14 du ventilateur 1, via un ou plusieurs conduits de gaz 23, de manière à introduire dans le flux d'air circulant dans le circuit de gaz 14 du ventilateur 1, le gaz additionnel riche en oxygène, par exemple de l'oxygène pur (i.e. teneur en oxygène 100% en vol.).

Les moyens de pilotage 12 commandent la vanne 24 contrôlant l'arrivée du gaz riche en oxygène dans le circuit de gaz 14, par exemple une électrovanne pilotée. Dans un autre mode de réalisation, l'alimentation en gaz riche en oxygène n'est pas contrôlée par la vanne 24 mais maitrisée par l'utilisateur qui en détermine la présence ou l'absence et les caractéristiques telles que débit et/ou pression.

L'introduction du gaz riche en oxygène se fait en un site d'adjonction 25 situé entre le premier capteur de débit 15 et le capteur de pression 16. Alternativement, l'introduction du gaz riche en oxygène peut se faire en un site d'adjonction situé soit en amont de la micro-soufflante 13, soit au niveau de la sortie d'air de la micro-soufflante 13 (non représenté).

Comme déjà mentionné, le ventilateur 1 comprend également une interface homme-machine ou IHM comprenant par exemple touches, boutons rotatifs ou translatifs ou analogues 21, permettant à l'utilisateur d'entrer des informations ou des consignes dans le ventilateur 1, ou d'opérer des choix, des validations ou des sélections dans des menus par exemple. L'IHM comprend en outre un écran d'affichage 22, tel un écran digital tactile, permettant non seulement d'afficher différentes informations, données, pictogrammes, graphiques etc... mais aussi une saisie ou entrée de données en vue de leur utilisation, notamment par les moyens de pilotage 20, ou aussi d'opérer des choix, des sélections, des validations... de paramètres, modes de fonctionnement ou autres.

Bien entendu, le ventilateur 1 peut comprend en outre des moyens d'alimentation en courant électrique (non montrés) comme un cordon et une prise de raccordement au secteur (110/220V), un transformateur de courant et/ou une batterie interne, alimentant les composants nécessitant du courant électrique pour fonctionner, notamment la micro-soufflante 13, notamment son moteur électrique, les moyens de pilotage 20, les capteurs 15-17, l'écran 22 de l'IHM ou tout autre composant.

Selon l'invention, les moyens de pilotage 12, en particulier leur microprocesseur, sont configurés pour estimer la résistance du patient P et du circuit patient 18, et en déduire un débranchement du circuit patient 18, à l'un ou l'autre des sites illustrés sur Fig. 1 à Fig. 3.

D'une façon générale, la résistance totale $R_{tot}(t)$ correspond à l'opposition à l'écoulement du débit gazeux se produisant dans le ventilateur 1, le circuit patient 18 et dans toute autre partie du trajet de gaz, y compris le patient P lui-même.

Lorsqu'il n'y a pas de débranchement du circuit patient 18, la résistance totale $R_{tot}(t)$ a obligatoirement une valeur minimale non-nulle due à la résistance du patient P lui-même et des éléments situés sur le trajet du gaz entre le ventilateur 1 et le patient P, tels que le circuit patient 18, l'interface respiratoire et les connectiques, tels que pièce en Y pour un circuit à double branche, conduits ou passages internes du ventilateur, et autres éléments.

A l'inverse, la résistance totale $R_{tot}(t)$ devient très faible, i.e. nulle ou quasi-nulle, lorsqu'un débranchement se produit puisque le flux de gaz s'échappe à l'atmosphère sans aucune opposition, c'est-à-dire sort librement du circuit patient 18 notamment.

Autrement dit, selon l'invention, on détecte tout débranchement du circuit patient 18 en utilisant le critère de la résistance totale $R_{tot}(t)$ mesurée et en déduisant qu'un tel débranchement s'est produit lorsque que le microprocesseur détermine que la résistance totale devient inférieure à un seuil donné, typiquement moins de 0,080 mmH2O/(cL/min), préférentiellement d'environ 0,010 à 0,070 mmH2O/(cL/min).

Pour ce faire, le processeur des moyens de pilotage 12 est configuré pour estimer, i.e. calculer, la résistance totale $R_{tot}(t)$ du patient P et du circuit patient 18, notamment à partir de mesures opérées par les capteurs de débit 15, 16 et le capteur de pression 17.

Plus précisément, en partant de l'équation de mouvement appliquée au patient, on a :

$$P_{prox}(t) = R_{patient}Q(t) + P_{musc}(t) + \frac{V(t)}{C}$$

où :

- $P_{prox}(t)$ est la pression proximale, i.e. au début des voies aériennes du patient,
- $P_{musc}(t)$ est la pression générée par l'effort musculaire du patient,
- $R_{patient}$ est la résistance du système respiratoire du patient,
- $Q(t)$ est le débit mesuré par le capteur de débit 17 de Fig. 2,
- $V(t)$ est le volume courant délivré au patient à chaque instant,
- $C$ est la compliance du système respiratoire du patient

En considérant que l'effort musculaire du patient sert majoritairement à compenser sa compliance, i.e.

$$\frac{V(t)}{c} = -P_{musc}(t)$$

, on obtient :

$$R_{pat} = \frac{P_{prox}(t)}{Q(t)}$$

Or, la pression patient $P_{prox}$ est estimée à partir de la pression et du débit amont mesurés par les capteurs de débit 17 et de pression 16. En prenant en compte la perte de charge, c'est-à-dire la résistance $R_{circuit}$ qui dépend du circuit patient 2, générée par le circuit patient 2, on a :

$$P_{machine}(t) - P_{prox}(t) = R_{circuit} \cdot Q(t)$$

Où :

- $P_{machine}$ est la pression amont mesurée par le capteur 16 de la Fig. 2,
- Q est le débit amont mesuré par le capteur 17 de la Fig. 2,
- $P_{prox}$ est la pression proximale, i.e. patient,
- $R_{circuit}$ la résistance, i.e. la perte de charge, générée par le circuit patient 2.

On obtient alors l'équation suivante :

$$R_{tot}(t) = R_{patient}(t) + R_{circuit}(t) = \frac{P_{machine}(t)}{Q(t)}$$

où :

- $R_{tot}(t)$ est la résistance totale appliquée au débit gazeux entre la sortie du ventilateur et les alvéoles pulmonaires du patient, c'est-à-dire incluant le circuit patient, le patient et tous les autres éléments ou composants (e.g. passages de gaz, pièce en Y, interface respiratoire...) pouvant présenter une résistance au débit de gaz,
- $R_{patient}(t)$ est la résistance au flux/débit gazeux des voies aériennes du patient,
- $R_{circuit}(t)$ est la résistance au flux/débit gazeux du circuit de ventilation, i.e. tuyau flexible et autres connectiques,
- $P_{machine}(t)$ est la pression à la sortie du ventilateur, par exemple mesurée par le capteur 16 de la Fig. 2, et
- $Q(t)$ est le débit délivré en sortie du ventilateur et fourni au patient, par exemple mesuré par le capteur 17 de la Fig. 2.

Cette équation est mise en œuvre dans le processeur des moyens de pilotage 12 pour calculer la résistance totale $R_{tot}(t)$.

Ensuite, le processeur compare la résistance $R_{tot}(t)$ calculée à une valeur-seuil de résistance donnée, à savoir une valeur minimale de résistance $R_{min}$, afin de détecter un problème de débranchement du circuit patient 18, lorsque la résistance calculée devient inférieure ou égale à ladite valeur minimale de résistance $R_{min}$, c'est-à-dire lorsque le processeur détermine que : $R_{tot}(t) \leq R_{min}$, avec de préférence : $R_{tot}(t) < R_{min}$.

La valeur-seuil minimale de résistance $R_{min}$ peut dépendre du patient P traité car le fonctionnement des poumons dépend notamment de l'âge du patient. Il est donc avantageux de pouvoir fixer une valeur-seuil minimale de résistance $R_{min}$ différente selon le patient P traité.

A cette fin, l'IHM est configurée pour permettre à l'utilisateur, tel un personnel soignant, tel un médecin ou analogue, de sélectionner une catégorie de patient, à savoir adulte, enfant ou nourrisson, et donc de pouvoir sélectionner ou fixer la valeur-seuil minimale de résistance $R_{min}$ la plus adéquate pour le patient considéré.

Le choix peut se faire via les moyens de sélection 21, tels un ou des organes de sélection 21 manuelle, tels que boutons, touches ou analogues, et/ou l'écran 22, notamment via une (ou des) touche(s) tactiles affichées à l'écran 22.

La valeur-seuil minimale de résistance $R_{min}$ peut être soit un paramètre entré par l'utilisateur, i.e. personnel soignant, soit être sélectionné parmi des choix affichés à l'écran, soit être une valeur fixée par défaut dès que la catégorie de patient a été choisie.

Typiquement, la valeur-seuil minimale de résistance ($R_{min}$) est comprise entre 0.010 et 0,080 mmH2O/(cL/min) selon le ventilateur, le circuit patient et le patient considérés, par exemple moins de 0,040 mmH2O/(cL/min).

Ainsi, par exemple, on peut utiliser les valeurs-seuils minimales de résistance ($R_{min}$) suivantes selon la catégorie de patient sélectionnée :

- Adulte : $R_{min}$ = 0.012 mmH2O/(cL/min),
- Enfant : $R_{min}$ = 0.020 mmH2O/(cL/min),
- Nourrisson : $R_{min}$ = 0.020 mmH2O/(cL/min).

Si la résistance $R_{tot}(t)$ calculée par le processeur devient inférieure à ce seuil $R_{min}$ pendant un temps de confirmation donné, par exemple pendant une durée de 3 à 5 secondes, le processeur détermine qu'un débranchement a eu lieu et déclenche alors une alarme sonore et/ou visuelle, par exemple un message d'alarme s'affichant sur l'écran 22 de l'IHM, l'allumage d'un dispositif d'alerte lumineux, tels une ou des LEDs par exemple, et/ou un signal sonore.

Le temps de confirmation est préférentiellement une durée préfixée et mémorisée soit par le microprocesseur lui-même, soit dans une mémoire de stockage portée par la carte électronique, ou sur un autre support informatique.

Préférentiellement, le processeur peut être aussi configuré pour détecter un rebranchement en résistance lorsqu'il détermine que R(t) > Rmin. Le processeur considère alors que le circuit est rebranché selon le critère de résistance. En réponse à cette détection du rebranchement, le processeur va stopper l'alarme que le processeur a déclenché après détection du débranchement.

D'une façon générale, un ventilateur médical selon l'invention permet de délivrer une aide respiratoire, c'est-à-dire une ventilation artificielle, à un patient souffrant de troubles ou insuffisances respiratoires plus ou moins sévères, pouvant résulter de différentes pathologies ou analogues, en particulier à un patient ventilé en milieu hospitalier, pendant plusieurs jours, même plusieurs semaines ou plusieurs mois.

**Revendications**

1. Ventilateur médical (1) comprenant :

    - un circuit de gaz interne (14) pour acheminer du gaz comprenant une sortie de gaz (26),
    - au moins un capteur de débit (15, 17) et au moins un capteur de pression (16) agencés sur le circuit de gaz interne (14) pour opérer des mesures de débit et de pression au sein dudit circuit de gaz interne (14),
    - des moyens de pilotage (12) à au moins un microprocesseur configurés pour traiter les mesures de débit et de pression opérées par lesdits capteurs de débit (15, 17) et de pression (16),
    - un circuit patient (18) raccordé fluidiquement à la sortie de gaz (26) du circuit de gaz interne (14), et
    - une interface respiratoire (19) raccordée fluidiquement au circuit patient (18),

    **caractérisé en ce que** les moyens de pilotage (12) sont configurés pour :

    a) déterminer une valeur de résistance $R_{tot}(t)$ à partir d'au moins des mesures de débit et de pression opérées par lesdits capteurs de débit (15, 17) et de pression (16),
    b) comparer la valeur de résistance $R_{tot}(t)$ déterminée à une valeur de résistance minimale $R_{min}$ donnée,
    c) déterminer un débranchement du circuit patient (18) ou de l'interface respiratoire (19) lorsque la valeur de résistance $R_{tot}(t)$ est inférieure ou égale à la valeur de résistance minimale $R_{min}$ donnée, et
    d) déclencher une alarme lorsqu'un débranchement du circuit patient (18) ou de l'interface respiratoire (19) est déterminé.

2. Ventilateur selon la revendication 1, **caractérisé en ce qu'**il comprend des moyens de fixation ou de sélection de résistance minimale pour fixer ou sélectionner une valeur de résistance minimale $R_{min}$.

3. Ventilateur selon l'une des revendications 1 ou 2, **caractérisé en ce qu'**il comprend des moyens de sélection de catégorie de patient (21, 22) permettant de sélectionner une catégorie de patient choisie parmi adulte, enfant et nourrisson, ladite sélection de catégorie de patient conduisant à une sélection automatique d'une valeur de résistance minimale $R_{min}$.

4. Ventilateur selon la revendication 1, **caractérisé en ce que** les moyens de pilotage (12) sont configurés pour calculer la valeur de résistance $R_{tot}(t)$ à partir de la formule suivante :

$$R_{tot}(t) = R_{patient}(t) + R_{circuit}(t) = \frac{P_{machine}(t)}{Q(t)}$$

où :

- $R_{tot}(t)$ est la résistance totale appliquée au débit gazeux entre la sortie du ventilateur et les alvéoles pulmonaires du patient,
- $R_{patient}(t)$ est la résistance des voies aériennes du patient,
- $R_{circuit}(t)$ est la résistance du circuit de ventilation, i.e. tuyau flexible et autres connectiques,
- $P_{machine}$ est la pression du gaz en sortie du ventilateur, et
- $Q(t)$ est le débit de gaz en sortie du ventilateur et fourni au patient.

5. Ventilateur selon la revendication 1, **caractérisé en ce que** les moyens de pilotage (12) comprennent au moins un microprocesseur agencé sur une carte électronique.

6. Ventilateur selon la revendication 3, **caractérisé en ce que** les moyens de sélection de catégorie de patient (21, 22) comprennent un organe de sélection manuelle (21) et/ou un écran d'affichage (22).

7. Ventilateur selon la revendication 1, **caractérisé en ce que** le circuit patient (18) comprend un ou deux conduits de gaz agencés en parallèle.

8. Ventilateur selon la revendication 1, **caractérisé en ce que** le circuit patient (18) est relié directement à interface respiratoire (19) ou indirectement via une pièce de raccordement, typiquement une pièce en Y.

9. Ventilateur selon la revendication 1, **caractérisé en ce que** la source de gaz (13) est une micro-soufflante motorisée.

10. Ventilateur selon la revendication 1, **caractérisé en ce que** les moyens de pilotage (12) sont configurés pour déterminer un débranchement du circuit patient (18) ou de l'interface respiratoire (19) lorsque la valeur de résistance $R_{tot}(t)$ est inférieure ou égale à la valeur de résistance minimale $R_{min}$ donnée (i.e. $R_{min} \geq R_{tot}(t)$) pendant un temps de confirmation donné.

11. Ventilateur selon la revendication 10, **caractérisé en ce que** le temps de confirmation donné a une durée de moins de 10 secondes.

12. Ventilateur selon l'une des revendications 2 ou 3, **caractérisé en ce que** les moyens de fixation ou de sélection de résistance minimale sont configurés pour fixer ou sélectionner une valeur de résistance minimale ($R_{min}$) comprise entre 0.010 et 0,080 mmH2O/(cL/min).

13. Ventilateur selon la revendication 12, **caractérisé en ce que** la valeur de résistance minimale ($R_{min}$) comprise entre 0.010 et 0,070 mmH2O/(cL/min).

14. Ventilateur selon la revendication 1, **caractérisé en ce qu'**il comprend en outre des moyens de mémorisation configurés pour mémoriser au moins une valeur de résistance minimale ($R_{min}$).

15. Ventilateur selon l'une des revendications 2, 3, 12, 13 ou 14, **caractérisé en ce que** la valeur de résistance minimale ($R_{min}$) est inférieure ou égale à 0,040 mmH2O/(cL/min).

**Patentansprüche**

1. Medizinisches Beatmungsgerät (1), umfassend:

   - einen internen Gaskreis (14) zum Befördern des Gases, der einen Gasauslass (26) umfasst,
   - mindestens einen Durchflussmengensensor (15, 17) und mindestens einen Drucksensor (16), die an dem internen Gaskreis (14) angeordnet sind, um Durchflussmengen- und Druckmessungen in dem internen Gaskreis (14) vorzunehmen,
   - Ansteuerungsmittel (12) mit mindestens einem Mikroprozessor, die dazu ausgelegt sind, die von den Durchflussmengensensoren (15, 17) und Drucksensoren (16) vorgenommenen Durchflussmengen- und Druckmessungen zu verarbeiten,
   - einen Patientenkreis (18), der fluidisch an den Gasauslass (26) des internen Gaskreises (14) angeschlossen ist, und

- eine Atemschnittstelle (19), die fluidisch an den Patientenkreis (18) angeschlossen ist,

**dadurch gekennzeichnet, dass** die Ansteuerungsmittel (12) dazu ausgelegt sind:

a) einen Widerstandswert $R_{tot}(t)$ ausgehend von den von den Durchflussmengensensoren (15, 17) und Drucksensoren (16) vorgenommenen Durchflussmengen- und Druckmessungen zu bestimmen,
b) den bestimmten Widerstandswert $R_{tot}(t)$ mit einem gegebenen minimalen Widerstandswert $R_{min}$ zu vergleichen,
c) eine Trennung des Patientenkreises (18) oder der Atemschnittstelle (19) zu bestimmen, wenn der Widerstandswert $R_{tot}(t)$ kleiner oder gleich dem gegebenen minimalen Widerstandswert $R_{min}$ ist, und
d) einen Alarm auszulösen, wenn eine Trennung des Patientenkreises (18) oder der Atemschnittstelle (19) bestimmt wird.

2. Beatmungsgerät nach Anspruch 1, **dadurch gekennzeichnet, dass** es Mittel zum Festlegen oder Auswählen eines minimalen Widerstands umfasst, um einen minimalen Widerstandswert $R_{min}$ festzulegen oder auszuwählen.

3. Beatmungsgerät nach einem der Ansprüche 1 oder 2, **dadurch gekennzeichnet, dass** es Mittel zum Auswählen einer Patientenkategorie (21, 22) umfasst, die es ermöglichen, eine unter Erwachsener, Kind und Säugling gewählte Patientenkategorie auszuwählen, wobei das Auswählen der Patientenkategorie zu einem automatischen Auswählen eines minimalen Widerstandswerts $R_{min}$ führt.

4. Beatmungsgerät nach Anspruch 1, **dadurch gekennzeichnet, dass** die Ansteuerungsmittel (12) dazu ausgelegt sind, den Widerstandswert $R_{tot}(t)$ ausgehend von der folgenden Formel zu berechnen:

$$R_{tot}(t) = R_{Patient}(t) + R_{Kreis}(t) = \frac{P_{Gerät}(t)}{Q(t)}$$

worin:

■ $R_{tot}(t)$ der Gesamtwiderstand ist, der auf die Gasdurchflussmenge zwischen dem Auslass des Beatmungsgeräts und den Lungenbläschen des Patienten angewandt wird,
■ $R_{patient}(t)$ der Widerstand der Atemwege des Patienten ist,
■ $R_{Kreis}(t)$ der Widerstand des Beatmungsgerätkreises ist, d. h. Schlauch und sonstige Anschlüsse,
■ $P_{Gerät}$ der Druck des Gases am Auslass des Beatmungsgeräts ist, und
■ $Q(t)$ die Gasdurchflussmenge am Auslass des Beatmungsgeräts ist, die an den Patienten abgegeben wird.

5. Beatmungsgerät nach Anspruch 1, **dadurch gekennzeichnet, dass** die Ansteuerungsmittel (12) mindestens einen Mikroprozessor umfassen, der auf einer Elektronikkarte angeordnet ist.

6. Beatmungsgerät nach Anspruch 3, **dadurch gekennzeichnet, dass** die Mittel zum Auswählen einer Patientenkategorie (21, 22) ein Element zum manuellen Auswählen (21) und/oder einen Anzeigebildschirm (22) umfassen.

7. Beatmungsgerät nach Anspruch 1, **dadurch gekennzeichnet, dass** der Patientenkreis (18) eine oder zwei parallel angeordnete Gasleitungen umfasst.

8. Beatmungsgerät nach Anspruch 1, **dadurch gekennzeichnet, dass** der Patientenkreis (18) mit der Atemschnittstelle (19) direkt verbunden ist oder indirekt über ein Anschlussstück, typischerweise ein Y-Stück.

9. Beatmungsgerät nach Anspruch 1, **dadurch gekennzeichnet, dass** die Gasquelle (13) ein motorisiertes Mikrogebläse ist.

10. Beatmungsgerät nach Anspruch 1, **dadurch gekennzeichnet, dass** die Ansteuerungsmittel (12) dazu ausgelegt sind, eine Trennung des Patientenkreises (18) oder der Atemschnittstelle (19) zu bestimmen, wenn der Widerstandswert $R_{tot}(t)$ während einer gegebenen Bestätigungszeit kleiner oder gleich dem gegebenen minimalen Widerstandswert $R_{min}$ ist (d. h. $R_{min} \geq R_{tot}(t)$).

11. Beatmungsgerät nach Anspruch 10, **dadurch gekennzeichnet, dass** die gegebene Bestätigungszeit eine Dauer von weniger als 10 Sekunden hat.

12. Beatmungsgerät nach einem der Ansprüche 2 oder 3, **dadurch gekennzeichnet, dass** die Mittel zum Festlegen oder Auswählen eines minimalen Widerstands dazu ausgelegt sind, einen minimalen Widerstandswert ($R_{min}$) zwischen 0,010 und 0,080 mmH2O/(cL/min) festzulegen oder auszuwählen.

13. Beatmungsgerät nach Anspruch 12, **dadurch gekennzeichnet, dass** der Wert des minimalen Widerstands ($R_{min}$) zwischen 0,010 und 0,070 mmH2O/(cL/min) liegt.

14. Beatmungsgerät nach Anspruch 1, **dadurch gekennzeichnet, dass** es ferner Speichermittel umfasst, die dazu ausgelegt sind, mindestens einen minimalen Widerstandswert ($R_{min}$) zu speichern.

15. Beatmungsgerät nach einem der Ansprüche 2, 3, 12, 13 oder 14, **dadurch gekennzeichnet, dass** der Wert des minimalen Widerstands ($R_{min}$) kleiner als oder gleich 0,040 mmH2O/(cL/min) ist.

## Claims

1. Medical ventilator (1) comprising:

   - an internal gas circuit (14) for conveying gas, comprising a gas outlet (26),
   - at least one flowrate sensor (15, 17) and at least one pressure sensor (16), which are arranged on the internal gas circuit (14) in order to perform flowrate and pressure measurements within said internal gas circuit (14),
   - control means (12) with at least one microprocessor, which are configured to process the flowrate and pressure measurements performed by said flowrate (15, 17) and pressure (16) sensors,
   - a patient circuit (18) fluidically connected to the gas outlet (26) of the internal gas circuit (14), and
   - a respiratory interface (19) fluidically connected to the patient circuit (18),

   **characterized in that** the control means (12) are configured to:

   a) determine a resistance value $R_{tot}(t)$ from at least flowrate and pressure measurements performed by said flowrate (15, 17) and pressure (16) sensors,
   b) compare the determined resistance value $R_{tot}(t)$ against a given minimum resistance value $R_{min}$,
   c) determine a disconnection of the patient circuit (18) or of the respiratory interface (19) when the resistance value $R_{tot}(t)$ is less than or equal to the given minimum resistance value $R_{min}$, and
   d) trigger an alarm when a disconnection of the patient circuit (18) or of the respiratory interface (19) is determined.

2. Ventilator according to Claim 1, **characterized in that** it comprises minimum resistance setting or selection means for setting or selecting a minimum resistance value $R_{min}$.

3. Ventilator according to either of Claims 1 and 2, **characterized in that** it comprises patient category selection means (21, 22) for selecting a patient category chosen from adult, child and infant, said patient category selection leading to an automatic selection of a minimum resistance value $R_{min}$.

4. Ventilator according to Claim 1, **characterized in that** the control means (12) are configured to calculate the resistance value $R_{tot}(t)$ from the following formula:

$$R_{tot}(t) = R_{patient}(t) + R_{circuit}(t) = \frac{P_{machine}(t)}{Q(t)}$$

where:

   ■ $R_{tot}(t)$ is the total resistance applied to the gas flowrate between the ventilator outlet and the pulmonary alveoli of the patient,
   ■ $R_{patient}(t)$ is the airway resistance of the patient,
   ■ $R_{circuit}(t)$ is the resistance of the ventilation circuit, i.e. flexible hose and other connections,
   ■ $P_{machine}$ is the pressure of the gas leaving the ventilator, and

■ Q(t) is the gas flowrate leaving the ventilator and delivered to the patient.

5. Ventilator according to Claim 1, **characterized in that** the control means (12) comprise at least one microprocessor arranged on an electronic card.

6. Ventilator according to Claim 3, **characterized in that** the patient category selection means (21, 22) comprise a manual selection member (21) and/or a display screen (22).

7. Ventilator according to Claim 1, **characterized in that** the patient circuit (18) comprises one or two gas ducts arranged in parallel.

8. Ventilator according to Claim 1, **characterized in that** the patient circuit (18) is connected directly to the respiratory interface (19) or indirectly via a connecting piece, typically a Y-piece.

9. Ventilator according to Claim 1, **characterized in that** the gas source (13) is a motorized micro-blower.

10. Ventilator according to Claim 1, **characterized in that** the control means (12) are configured to determine a disconnection of the patient circuit (18) or of the respiratory interface (19) when the resistance value $R_{tot}(t)$ is less than or equal to the given minimum resistance value $R_{min}$ (i.e. $R_{min} \geq R_{tot}(t)$) during a given confirmation time.

11. Ventilator according to Claim 10, **characterized in that** the given confirmation time has a duration of less than 10 seconds.

12. Ventilator according to either of Claims 2 and 3, **characterized in that** the minimum resistance setting or selection means are configured to set or select a minimum resistance value ($R_{min}$) of between 0.010 and 0.080 mmH2O/(cL/-min).

13. Ventilator according to Claim 12, **characterized in that** the minimum resistance value ($R_{min}$) is between 0.010 and 0.070 mmH2O/(cL/min).

14. Ventilator according to Claim 1, **characterized in that** it further comprises storage means configured to store at least one minimum resistance value ($R_{min}$).

15. Ventilator according to one of Claims 2, 3, 12, 13 and 14, **characterized in that** the minimum resistance value ($R_{min}$) is less than or equal to 0.040 mmH2O/(cL/min) .

Fig. 1

Fig. 2

Fig. 3

Fig. 4

## RÉFÉRENCES CITÉES DANS LA DESCRIPTION

**Documents brevets cités dans la description**

- WO 8201654 A

- WO 8201654 A1